(19) Europäisches Patentamt
European Patent Office
Office européen des brevets

(11) **EP 4 000 501 A1**

(12) **EUROPEAN PATENT APPLICATION**
published in accordance with Art. 153(4) EPC

(43) Date of publication:
**25.05.2022 Bulletin 2022/21**

(21) Application number: **20840504.3**

(22) Date of filing: **10.07.2020**

(51) International Patent Classification (IPC):
***A61B 3/10*** *(2006.01)*

(52) Cooperative Patent Classification (CPC):
**A61B 3/10**

(86) International application number:
**PCT/JP2020/027126**

(87) International publication number:
**WO 2021/010345 (21.01.2021 Gazette 2021/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.07.2019 JP 2019131446**

(71) Applicant: **Nidek Co., Ltd.**
**Gamagori-shi,**
**Aichi, 443-0038 (JP)**

(72) Inventor: **HIGUCHI Yukihiro**
**Gamagori-shi Aichi 443-0038 (JP)**

(74) Representative: **Hoefer & Partner Patentanwälte mbB**
**Pilgersheimer Straße 20**
**81543 München (DE)**

(54) **OPHTHALMOLOGIC IMAGING APPARATUS**

(57)     An ophthalmologic imaging apparatus 1 including at least an SLO optical system 300, a focusing lens 63, a light-receiving element 68, and a control unit 70. The SLO optical system 300 includes: an irradiation optical system that irradiates light on to an eye being examined E; and a light-receiving optical system that receive return light from the eye being examined, by using a light-receiving element 68. A frontal image of the fundus of the eye being examined is captured on the basis of signals from the light-receiving element 68. The focusing lens 63 is arranged on the optical path of the SLO optical system 300 and is controlled in order to adjust the focal position of the SLO optical system 300, relative to the eye being examined E. The gain relative to the signals from the light-receiving element 68 can be controlled. The control unit 70 controls the focusing lens on the basis of sequentially acquired frontal images of the fundus and, at the same time or prior to that, adjusts the gain on the basis of sequentially obtained frontal images.

FIG. 5

EP 4 000 501 A1

**Description**

TECHNICAL FIELD

The present disclosure relates to an ophthalmologic imaging apparatus.

BACKGROUND ART

In recent years, optical coherence tomography (OCT) has been widely used in the field of ophthalmology. OCT captures a tomographic image of a subject eye.

Further, as a method for optimizing a focus position in an ophthalmologic imaging apparatus, a method using an image of the subject eye acquired by an imaging optical system is known. In this method, a predetermined evaluation value is acquired from an image obtained at each time when the focus position is changed. Then, the focus position is adjusted to a position where the evaluation value peaks.

Patent Literature 1 discloses an apparatus in which a focus position of a front imaging optical system that captures a front image of a fundus is adjusted by using the above-described method. Further, Patent Literature 1 discloses an apparatus in which a focus position of an OCT optical system is adjusted in conjunction with the focus position of the front imaging optical system.

CITATION LIST

PATENT LITERATURE

Patent Literature 1: JP-A-2013-188316

SUMMARY OF INVENTION

However, when an excellent image of the subject eye cannot be obtained, for example, because the subject eye is a diseased eye, a case is considered where the focus position is not properly adjusted by the above-described method.

In view of the above-described problems, a technical subject of the present disclosure is to provide an ophthalmologic imaging apparatus in which the focus position is excellently adjusted based on an image of the subject eye.

According to a first aspect of the present disclosure, there is provided an ophthalmologic imaging apparatus including:

an imaging optical system including an irradiation optical system that irradiates a subject eye with light and a light receiving optical system that receives return light from the subject eye by a light receiving element, and capturing an image of the subject eye based on a signal from the light receiving element;
a first optical device disposed in an optical path of the imaging optical system and controlled to adjust a focusing position of the imaging optical system with respect to the subject eye;
a second optical device controlled to adjust at least one imaging condition among an amount of the illumination light, a gain for the signal from the light receiving element, and an exposure time; and
a control means for controlling the first optical device based on a sequentially-acquired image of the subject eye, and for controlling the second optical device based on the sequentially-acquired image of the subject eye simultaneously with or before controlling the first optical device.

According to the present disclosure, the focus position is excellently adjusted based on the front image.

BRIEF DESCRIPTION OF DRAWINGS

[FIG. 1] FIG. 1 is a view illustrating an optical system and a control system of a fundus imaging apparatus of the present example.
[FIG. 2] FIG. 2 is a view illustrating an example of an observation image acquired by an observation optical system.
[FIG. 3] FIG. 3 is a view illustrating an example of a tomographic image acquired by an OCT optical system.
[FIG. 4] FIG. 4 is a flowchart illustrating an operation flow of the apparatus in the example.
[FIG. 5] FIG. 5 is a flowchart for describing a flow of optimization control in more detail.

[FIG. 6] FIG. 6 is a view illustrating an example of a differential histogram of an SLO image.

[FIG. 7] FIG. 7 is an example of a graph illustrating a relationship between an evaluation value C related to an image formation state and a focus position.

[FIG. 8] FIG. 8 is an example of a graph illustrating a relationship between the evaluation value C related to the image formation state and the focus position in a case where a subject eye under conditions different from that of FIG. 6 is captured.

[FIG. 9] FIG. 9 is a graph illustrating a relationship between an optical path length difference and an evaluation value B.

DESCRIPTION OF EMBODIMENTS

Hereinafter, an embodiment of an ophthalmologic imaging apparatus according to the present disclosure will be described. In the following embodiments, the ophthalmologic imaging apparatus is a fundus imaging apparatus unless otherwise specified.

"Overview"

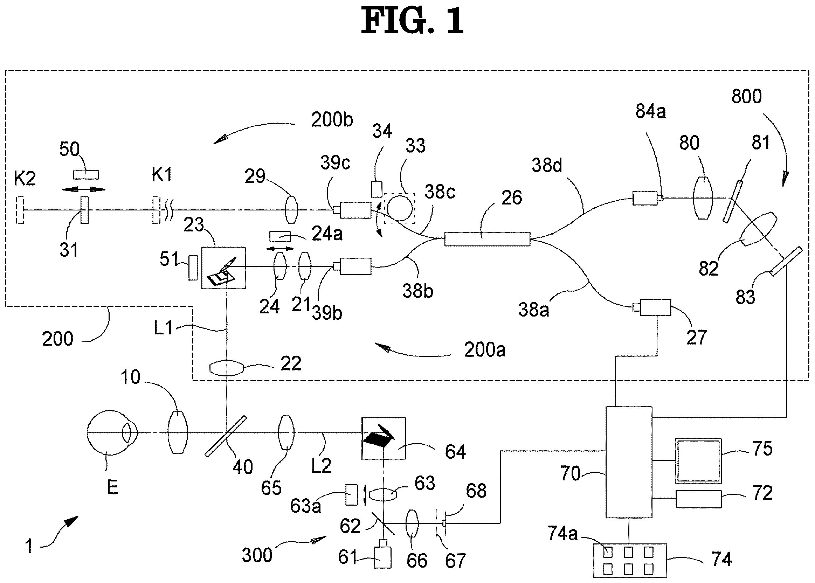

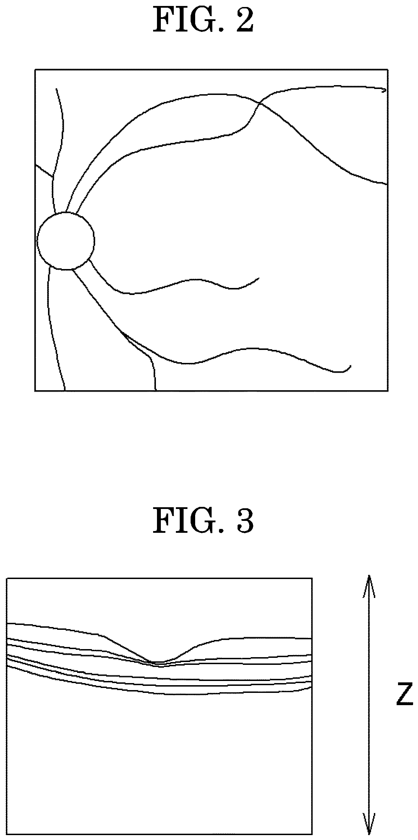

The ophthalmologic imaging apparatus (for example, refer to FIG. 1) captures at least a fundus image of a subject eye (for example, refer to FIG. 2). In the present embodiment, the ophthalmologic imaging apparatus includes at least an imaging optical system, a first optical device (focus adjusting unit), a second optical device, and a control unit. Furthermore, the ophthalmologic imaging apparatus may include a second optical system. Each of the first optical device and the second optical device may be a part of the imaging optical system. The optical device in the present embodiment is a general term for apparatuses that realize recording and transmission of information by using light in the ophthalmologic imaging apparatus, and examples thereof include a lens, a mirror, a fiber, a diaphragm, a filter, a scanner, a light source, a light receiving element, and the like.

<Imaging Optical System>

As described below as an example, the imaging optical system of the present embodiment may be a front imaging optical system. The front imaging optical system captures a front image (here, a front image of the fundus) of the subject eye as an image of the subject eye. The imaging optical system may be an observation optical system that acquires an observation image of the fundus as a front image. The observation image is used for various adjustments of the apparatus. The observation image may be acquired by invisible light, such as infrared light, as illumination light (also referred to as observation light), and the front image may be acquired as a moving image. The imaging optical system may be a non-scanning type optical system or a scanning type optical system.

The imaging optical system includes an irradiation optical system and a light receiving optical system. The irradiation optical system irradiates the fundus of the subject eye with light. Further, the light receiving optical system has a light receiving element and receives return light from the fundus by the light receiving element. A front image of the fundus is captured based on a signal from the light receiving element.

<First Optical Device>

The first optical device is disposed in an optical path of the imaging optical system and is controlled to adjust the focus position (focusing position) of the imaging optical system with respect to the subject eye. The first optical device is the focus adjusting unit in the present embodiment. The focus adjusting unit may include, for example, a moving lens, a variable focus lens such as a liquid crystal lens, or an optical system capable of changing the optical path length. The optical system capable of changing the optical path length may be, for example, one or a plurality of lenses, mirrors, or a combination thereof.

<Second Optical Device>

The second optical device is controlled to adjust at least one imaging condition among an amount of light emitted from the imaging optical system to the subject eye, a gain on the signal from the light receiving element, and the exposure time. The second optical device may be, for example, a light source of the imaging optical system or the light receiving element (including a gain control circuit). In a case where the imaging optical system is the scanning type optical system, an optical scanner may be used as the second optical device. The second optical device is not necessarily limited to these, and may be another optical device used for adjusting a condition related to intensity of light receiving signal.

<Control Unit>

The control unit (a control means in the present embodiment) is a processor that controls various operations of the ophthalmologic imaging apparatus. The control unit may include, for example, a CPU, a RAM, a ROM, and the like.

<Focus Position Adjustment Control>

The control unit of the present embodiment acquires front images at each of the plurality of focus positions while changing the focus position. In addition, the control unit acquires an evaluation value related to a focus state from each of the front images, and specifies an optimum focus position based on the evaluation value. Here, the quality of the front image is greatly affected by the image formation state (focus state) of the front image. That is, in a case where the subject is in focus, the quality of the front image is improved. In a case where the image is out of focus, the quality of the front image deteriorates. Here, for example, an evaluation value of image quality may be used as an example of the evaluation value related to the focus state. The position where the image quality is the best (that is, the position where the evaluation value is maximized) may be specified as the optimum focus position.

However, when the subject eye is a diseased eye such as a cataract eye, a change in the evaluation value of the image quality with respect to a change in the focus position becomes small, and as a result, there is a case where the optimum focus position cannot be properly specified. On the other hand, for example, it is effective that the imaging condition related to the second optical device is adjusted in advance such that a light receiving signal having a higher intensity can be obtained. Accordingly, an amount of change in the evaluation value with respect to the change in the focus position becomes large, and the position where the image quality is the best can be easily specified.

However, when the imaging condition related to the second optical device is adjusted in advance such that the light receiving signal having a higher intensity can be obtained, pixel values are likely to be saturated when imaging a normal eye. Accordingly, relationship between the evaluation value and actual focus state deviates, and a case is assumed where the optimum focus position cannot be properly specified based on the evaluation value. In this case, for example, compared to a case of imaging the diseased eye, it is more effective that the imaging condition related to the second optical device is adjusted in advance such that a light receiving signal having a small intensity can be obtained.

However, an examiner cannot always grasp characteristics such as whether or not the subject eye is a diseased eye in advance.

Here, for example, the control unit may simultaneously control each of the adjustment of the focus position and the adjustment of the imaging condition related to the second optical device, based on a sequentially-acquired front image. Accordingly, the optimum focus position is searched for while the imaging condition related to the second optical device is adjusted according to the subject eye such that a light receiving signal having an appropriate intensity can be obtained. Therefore, the optimum focus position can be more reliably specified regardless of the subject eye.

In this case, an initial value of the imaging condition related to the second optical device may be set to a value, at a part of a scope, such that a luminance value is saturated in a case where the focus position is changed with respect to the normal eye, for example. When the focus position is changed and the evaluation value becomes equal to or higher than a predetermined threshold value, the imaging condition of the second optical device may be changed to a level at which a light receiving signal with a lower intensity can be obtained. Accordingly, the evaluation value can be maximized while reducing the saturation of the luminance value while the focus position is changed in a predetermined range, and thus, the optimum focus position can be more reliably specified.

Further, for example, the imaging condition related to the second optical device may be adjusted based on the sequentially-acquired front image, and then the focus position adjustment control may be started. It is desirable that the imaging condition related to the second optical device is adjusted at the same time during the focus position adjustment control. However, the present disclosure is not necessarily limited thereto. For example, it is also considered that the imaging condition related to the second optical device is properly adjusted according to the characteristics of the subject eye, and thus, the adjustment control of the second optical device becomes unnecessary in the subsequent focus position adjustment control.

<Focus Control of Second Optical System>

The ophthalmologic imaging apparatus may include a second optical system. The second optical system irradiates at least a captured scope of the front image of the fundus with the second light. The second optical system may be an imaging optical system or a measuring optical system having a modality different from that of the imaging optical system, or may be an irradiation optical system that irradiates therapeutic light (therapeutic beam).

In the present embodiment, the focus position of the second optical system with respect to the fundus may be adjusted in conjunction with the focus position of the imaging optical system. In this case, the focus adjusting unit in the second optical system (hereinafter, also referred to as "second focus adjusting unit") may be a separate body from the focus adjusting unit in the imaging optical system. Both of the focus adjusting units may be mechanically interlocked (linked) or interlocked by simultaneous control such that the focus position in the second optical system and the focus position in the imaging optical system match each other. Further, the focus adjusting unit may be shared between the second optical system and the imaging optical system.

<Regarding Case of Applying OCT Optical System to Second Optical System>

The second optical system may be an OCT optical system for acquiring an OCT image (tomographic image, for example, refer to FIG. 3) in the captured scope of the front image. At this time, the captured scope of the OCT image may be narrower than the captured scope of the front image, may be equal to the captured scope of the front image, or may be wider than the captured scope of the front image. The OCT optical system acquires an OCT image based on a spectral interference signal between measurement light and reference light. The OCT optical system has at least a light splitter and a detector. The measurement light and the reference light are generated by splitting the light from the light source by the light splitter. Return light, of the measurement light, from the subject eye and the reference light are guided to the detector.

In the OCT optical system, an OPL is appropriately adjusted, and thus, an OCT image of the subject eye can be obtained based on the spectral interference signal. Here, the OPL is the optical path length difference between the measurement light and the reference light. In this case, the ophthalmologic imaging apparatus may further include an OPL adjusting unit (third optical device) for adjusting the OPL in the OCT optical system. Any of various configurations may be appropriately applied to the OPL adjusting unit. The OPL adjusting unit includes at least a driver (driving unit).

In a case where the second optical system is an OCT optical system, the control unit may simultaneously control the adjustment of the OPL simultaneously with the adjustment of the focus position and the adjustment of the imaging condition related to the second optical device. Accordingly, the focus position and the OPL in the OCT optical system can be adjusted to the optimum position in a shorter period of time.

The OPL adjustment performed at the same time as the adjustment of the focus position and the adjustment of the imaging condition related to the second optical device may be a rough adjustment. After the rough adjustment is completed, the OPL may be finely adjusted based on the OCT image obtained by the OCT optical system.

<Application to Anterior Eye Part>

In the above-described embodiment, the ophthalmologic imaging apparatus has been described as an apparatus that captures the fundus of the subject eye, but the present invention is not necessarily limited thereto. For example, the ophthalmologic imaging apparatus may obtain a front image of an anterior eye part of the subject eye by the imaging optical system. Even in this case, a certain effect can be obtained in shortening the adjustment time before imaging.

<Regarding Case of Applying OCT Optical System to Imaging Optical System>

Further, in the above-described description, a case where the imaging optical system is the front imaging optical system has been illustrated, but the imaging optical system may be an OCT optical system. The OCT optical system captures a tomographic image of the fundus as an image of the subject eye based on the spectral interference signal between the measurement light and the reference light. In a case of controlling the focus position in the OCT optical system based on the image information of a sequentially-acquired tomographic image, at the same time or before that, in the OCT optical system, by adjustment of the imaging condition related to the intensity of the light receiving signal based on the image information of the sequentially-acquired tomographic image, the focus position can be optimized more reliably.

"Example"

Next, an example of the present disclosure will be illustrated with reference to the drawings. In the description of the example, an axial direction of a subject eye E will be described as a Z direction, a horizontal direction will be described as an X direction, and a vertical direction will be described as a Y direction. A surface direction of the fundus may be considered as an XY direction.

The fundus imaging apparatus (OCT device) 1 of the present example is a type of an ophthalmologic imaging apparatus according to the embodiment. As illustrated in FIG. 1, the OCT device 1 according to the example includes an interference optical system (OCT optical system) 200, an observation optical system 300 (SLO optical system), and a control unit 70. The optical paths of the interference optical system 200 and the observation optical system 300 are coupled and branched by a dichroic mirror 40.

<OCT Optical System>

FIG. 1 illustrates an SD-OCT optical system as an example. However, the present disclosure is not necessarily limited thereto. The fundus imaging apparatus 1 may be, for example, SS-OCT or OCT based on other imaging principles.

The OCT optical system 200 includes at least a light source 27, a light splitter 26, a polarizer 33, and a spectroscopic optical system (spectrometer) 800. The light source 27 emits low coherent light. The light emitted from the light source 27 is split into the measurement light and the reference light by the light splitter 26. In the present example, a coupler (splitter) is used as the light splitter 26. The measurement light is guided to the fundus of the subject eye via a measuring optical system 200a, and the reference light is guided to a reference optical system 200b. Further, the polarizer 33 adjusts the polarization of the measurement light and the reference light. In FIG. 1, the polarizer 33 is disposed on a reference optical path. The reference light is reflected by a mirror 31 disposed on the reference optical path, and is incident on the spectroscopic optical system 800 in a state of being combined with the return light of the measurement light by the coupler 26. The spectroscopic optical system 800 disperses the return light and the reference light for each frequency (wavelength), and causes a detector 83 (in the present embodiment, the line sensor) to receive the dispersed light. The OCT image of the fundus (for example, a tomographic image, refer to FIG. 3) is acquired based on the spectral interference signal between the return light and the reference light.

An optical fiber 38b, a collimator lens 21, a focusing lens 24, a scanning unit (optical scanner) 23, a mirror 25, a relay lens 22, and a dichroic mirror 40, and an objective lens 10 are disposed on a measurement optical path between the light splitter 26 and the subject eye E.

In the present example, the focus adjusting unit of the OCT optical system 200 includes a focusing lens 24 and a driver 24a. The focusing lens 24 is displaced in an optical axis direction by the driver 24a, and thus, the focus position in the OCT optical system 200 is changed. As an example, the focusing lens 24 may be movable in the range of-12D to +12D.

The initial position of the focusing lens 24 is a position corresponding to an average eye refractive power of the subject eye (for example, a position corresponding to 0D). It is needless to say that a position before moving to the initial position may be used as the initial position. Further, the initial position may be set to be changed in any manner. Either a first movement limit position or a second movement limit position may be the initial position.

The scanning unit 23 is used to change an acquisition position of the OCT image on the fundus. The scanning unit 23 may be used to scan the measurement light two-dimensionally (XY directions) on the fundus (subject). The scanning unit 23 may include, for example, two optical scanners having different scanning directions. Each of the optical scanners may be a galvanometer mirror or another optical scanner.

The objective lens 10 forms a pivot point of the measurement light, and guides the measurement light to the fundus of the subject eye via the pivot point. With respect to the objective lens 10, the scanning unit 23 is disposed at a position conjugate with a pupil of the subject eye. Accordingly, the measurement light is pivoted around one point on the pupil according to a driving amount of the scanning unit 23.

In the present example, the OPL adjusting unit adjusts the OPL by changing the optical path length of the reference optical path. Accordingly, the captured scope in the depth direction of the OCT is adjusted according to the individual difference in an eye axis length. As an example, the OPL adjusting unit illustrated in FIG. 1 includes the mirror 31 and a driver 50, and the OPL is adjusted by the mirror 31 being displaced by the driver 50. However, the OPL adjusting unit is not necessarily limited thereto. For example, the OPL adjusting unit may adjust the OPL by changing the optical path length in the measurement optical path. In this case, for example, the OPL adjusting unit may integrally displace an end portion of the optical fiber 38b and the collimator lens 21 along the optical axis. Although the reference optical path is illustrated as a reflection type optical system in FIG. 1, the reference optical path is not necessarily limited thereto, and may be formed by a transmission type optical system.

<Observation Optical System>

In FIG. 1, an SLO optical system is illustrated as an example of the observation optical system 300. The observation optical system 300 may include at least an irradiation optical system and a light receiving optical system. The irradiation optical system irradiates the fundus with illumination light. The light receiving optical system receives the fundus reflected light by a light receiving element 68. Based on the output signal from the light receiving element 68, a front image (refer to FIG. 2) of the fundus is sequentially acquired as an observation image.

The observation optical system 300 further has a focus adjusting unit. The focus adjusting unit includes a focusing lens 63 and a driver 63a for driving the focusing lens 63.

As the light source 61, for example, a laser diode light source is used. In addition to the focusing lens 63, a scanning unit 64, a relay lens 65, and the objective lens 10 are disposed in an optical path that emits light (illumination light in the example) emitted from the light source 61 toward the subject eye E. Further, the scanning unit 64 is disposed at a position substantially conjugate with the pupil of the subject eye. The scanning unit 64 scans the light two-dimensionally on the fundus. The scanning unit 64 may include, for example, a combination of a polygon mirror and a galvano scanner.

Further, a beam splitter 62 is disposed between the light source 61 and the focusing lens 63. A condenser lens 66, a confocal aperture 67, and the light receiving element 68 are disposed in the reflection direction of the beam splitter 62.

The light from the light source 61 (illumination light in the example) passes through the beam splitter 62 and then reaches the scanning unit 64 via the focusing lens 63. The light that has passed through the scanning unit 64 passes through the dichroic mirror 40 via the relay lens 65, and then is applied to the fundus via the objective lens 10.

The fundus reflected light is guided back to the beam splitter 62 in the light projection path. The fundus reflected light is reflected by the beam splitter 62, and is received by the light receiving element 68 via the condenser lens 66 and the confocal aperture 67. A front image of the fundus is formed based on a light receiving signal from the light receiving element 68. The formed front image may be stored in the memory 72.

The dichroic mirror 40 has characteristics of reflecting the measurement light (for example, approximately $\lambda =$ 840 nm) emitted from the measurement light source 27 used in the OCT optical system 200, and transmitting the laser light (light having a wavelength different from that of the OCT light source 27, for example, approximately $\lambda =$ 780 nm) emitted from the light source 61 used in the observation optical system 300. In this case, the dichroic mirror 40 makes a measurement optical axis L1 of the OCT optical system 200 and a measurement optical axis L2 of the observation optical system 300 coaxial.

An optical fiber 38c is rotationally moved by being driven by the driver 34, and the movable range thereof is set. The optical fiber 38c can rotationally move from the first movement limit position (for example, 0°) to the second movement limit position (for example, 180°).

The optical fiber 38c is disposed at an intermediate position between the first movement limit position and the second movement limit position, and is not moved until after completion of the second automatic optical path length adjustment. Therefore, in the polarizer 33, the intermediate position is the initial position.

<Control System>

Next, a control system of the OCT device 1 will be described.

The control unit 70 of the OCT device 1 controls various operations in the OCT device 1. In addition, in the present example, the control unit 70 performs various image processing. That is, the control unit 70 also serves as an image processor. The control unit 70 may include, for example, a CPU, a RAM, a ROM, and the like.

Further, in the present example, the control unit 70 is connected to a monitor 75 and controls display of the monitor 75. Furthermore, the control unit 70 is connected to the memory 72, an operation unit 74, the drivers 24a, 30, 50, and 63a, and the like.

<Control Operation>

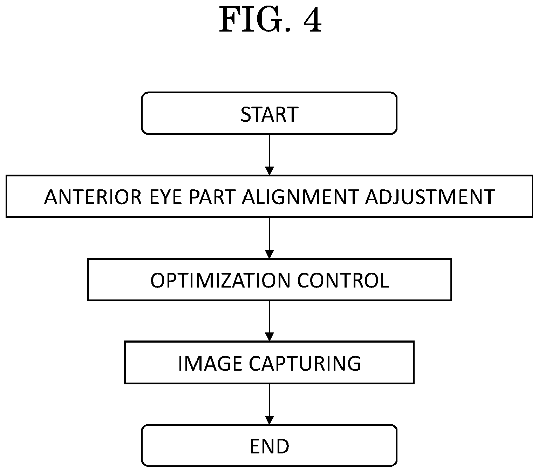

The operation of the apparatus in the present example will be described with reference to the flowchart of FIG. 4. The flowchart of FIG. 4 illustrates a flow from alignment to imaging.

<Alignment Adjustment

First, alignment of the apparatus with respect to the subject eye is performed. After having the examinee gaze at a fixation target in advance, the positional relationship between the subject eye and a measurement optical axis is adjusted based on the observation image of the anterior eye part captured by an anterior eye part observation camera which is not illustrated. For example, the center of the pupil of the subject eye and the measurement optical axis are adjusted to match each other. The alignment may be adjusted manually or automatically. At the position where the alignment adjustment is completed, a front image (SLO image) of the fundus can be acquired by the observation optical system 300.

After the alignment is completed, an acquisition of the observation image via the observation optical system 300 and a display of the observation image on the monitor 75 are started. At the same time, the control unit 70 acquires the OCT image sequentially via the OCT optical system 200.

<Optimization Control>

Next, optimization control of imaging condition is performed. By performing the optimization control, a region of the fundus desired by the examiner can be observed with high sensitivity and high resolution by the OCT optical system 200. In the present example, as an example of the optimization control in the OCT optical system 200, optical path length adjustment, focus adjustment, and polarization state adjustment (polarizer adjustment) are performed.

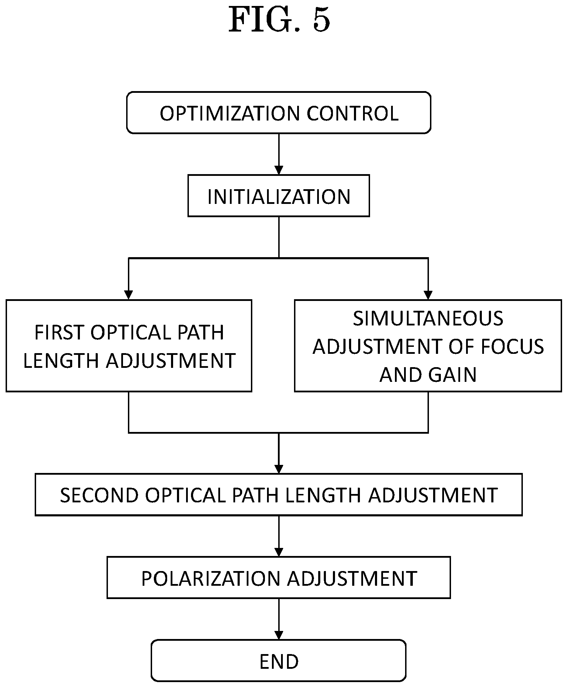

In the present example, the optimization control is started in response to the operation of an optimization start switch (optimize switch) 74a disposed in the operation unit 74 as a trigger. Hereinafter, an example of an operation flow in the optimization control will be described with reference to the flowchart of FIG. 5.

<Initialization>

First, the control unit 70 initializes the OPL and the focus position. For example, each of the position of the focusing lens 24 and the position of the mirror 31 is moved to a predetermined initial position (movement start position). In the present example, each initial position may be either upper limit or lower limit of the movable range.

After initialization, in the present example, the adjustment of the focus position and the adjustment of the first optical path length (a type of adjustment of the OPL) are controlled simultaneously.

<First Focus Adjustment>

In the first focus adjustment, the focus position in the observation optical system 300 is adjusted based on the observation image by the SLO image. In the present example, the gain of the observation image (SLO image) is automatically adjusted simultaneously. As a result of the first focus adjustment, it is sufficient when the quality of the SLO image is within an acceptable range as an observation image, and it is not necessary to adjust the focus position to a strict optimum position.

As an example, in the present example, information on a differential histogram of the SLO image is used as an evaluation value C. The differential histogram is a histogram based on a differential value of the pixel value obtained by differentiating the image data of the SLO image. For example, after the image data of the SLO image is converted into a contour image by applying a filter for edge extraction (for example, Laplacian conversion and SOBEL), the histogram of the contour image is acquired as the differential histogram.

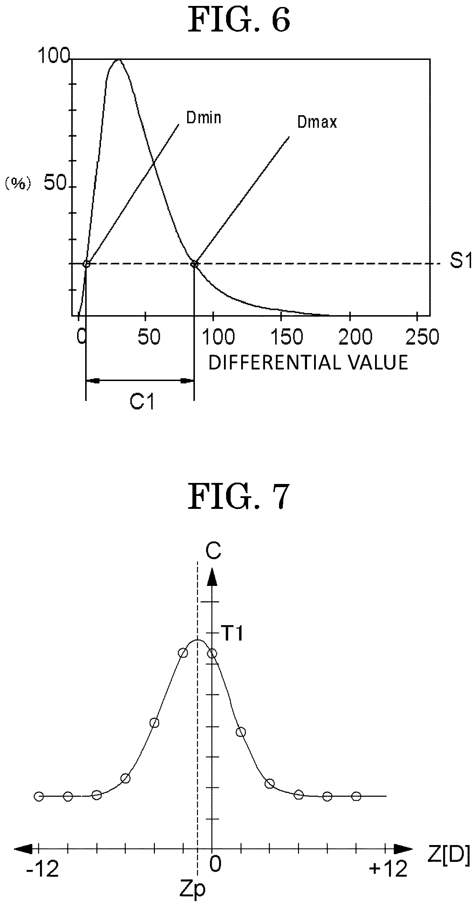

FIG. 6 is a view illustrating an example of the differential histogram. In FIG. 6, a horizontal axis is an absolute value d of the differential value (hereinafter, simply referred to as a differential value). FIG. 6 illustrates a case where the differential value d is represented by a value of 255 gradations from 1 to 254. A vertical axis is a value obtained by normalizing the number of pixels corresponding to each differential value. In the differential value having the largest number of corresponding pixels in the image, the number of the corresponding pixels is defined as 100%, and the number of pixels corresponding to each differential value is normalized. In the histogram illustrated in FIG. 6, the data of two end points (d = 0, d = 255) are excluded.

The differential histogram illustrates that the more proper the focus, the sharper the edge at the blood vessel region of the fundus. Therefore, the more appropriate the focus, the greater the number of pixels that take a large differential value. Meanwhile, the differential histogram illustrates a tendency for the edge to become dull as the focus position deviates from the optimum position. Accordingly, as the focus position deviates from the optimum position, the number of pixels that take a large differential value decreases.

In the present example, the evaluation value C of the image formation state (focus state) of the SLO image is calculated using the differential value having a certain number of pixels or more in the differential histogram. In the example illustrated in FIG. 6, 20% is set as an example of the threshold value S1.

The evaluation value C is expressed as follows in the present example.

$$C1 = Dmax - Dmin$$

Here, Dmax is the maximum value of the differential value having pixels equal to or higher than the threshold value, and Dmin is the maximum value of the differential value having pixels equal to or higher than the threshold value. As in the present example, by setting a threshold value S1 to approximately 20%, it is possible to accurately detect a change in the sharpness of the edge at the fundus blood vessel region, which occupies a small scope in the entire SLO image. It is needless to say that a value of approximately 20% is only an example of the threshold value S1. The threshold value S1 can be appropriately set within a range in which the evaluation value C properly changes with respect to a change in the image formation state while avoiding the influence of noise. Further, in the above, the maximum value Dmax itself of the differential value may be used as an evaluation value related to the image formation state.

<Moving Mode of Focusing Lens>

In the present example, the control unit 70 acquires the evaluation value C related to the focus state from the observation image acquired at each focus position, and specifies the optimum focus position based on the evaluation value C.

As an example, the focus position is moved in one direction from the initial position. For example, the position at -12D is set as the initial position, the position is moved in the plus direction in 2D steps, and the evaluation value C is acquired in each step up to +12D. It is needless to say that a case where an amount of change per step to 2D is an example, and the amount of change per step may be a value larger than 2D or a value smaller than 2D within a range in which the optimum focus position can be specified.

Here, the graph illustrated in FIG. 7 illustrates the relationship between the evaluation value C and the position Z of the focusing lens 63 in a case where the evaluation value C is acquired without saturation at each focus position. A position where the evaluation value C is maximized may be obtained as a target position of the focus position. Further, as illustrated in FIG. 7, for example, a scatter diagram in which the evaluation value C acquired in each step is plotted is subjected to curve approximation (an example of interpolation processing) by a function having a maximum value in a moving range of the focusing lens 63, and a peak position Zp of the evaluation value C on this curve may be obtained as the target position.

The control unit 70 moves the focusing lens 63 to the target position obtained as a result of the above processing. In this manner, the focusing lens 63 is adjusted in the first focus adjustment.

<Gain Adjustment and Method for Determining Focus Target Position based on Gain Adjustment>

For example, the gain value is important in acquiring the evaluation value C in the focus state as described above.

When the gain value is small, the change in the image quality evaluation value C with respect to the change in the focus position becomes small in a case where the subject eye is opacified or the like. As a result, the optimum focus position may not be properly specified based on the evaluation value C.

Further, when the gain value is large, it becomes easy to detect the optimum focus position in a case where the subject eye is opacified or the like, but in a case where the subject eye is not opacified or the like, the saturation of the luminance value tends to occur, and there is a case where the relationship between the evaluation value C and the actual focus state deviates. In the present example, in this case, saturation of the evaluation value C occurs.

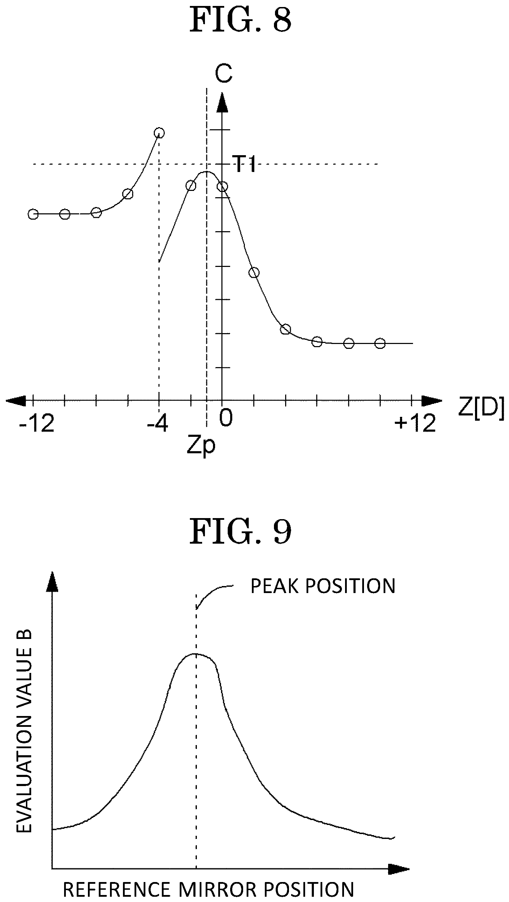

In view of the above points, in the present example, an initial value Gp of the gain is set (or adjusted) to a somewhat high value (however, the initial value Gp is a value smaller than a threshold value T1 described later). Further, the control unit 70 compares the evaluation value C and the threshold value T1 at the focus position of each step, and reduces the gain in a case where the evaluation value C exceeds the threshold value T1. An amount of decrease in gain at this time may be a constant value. Even when the gain is reduced, in a case where the evaluation value C still exceeds the threshold value T1, the gain may be repeatedly reduced. The number of times the gain has been reduced after the focus adjustment was started (here, the cumulative number of times, and hereinafter, referred to as a count value) may be counted. When the evaluation value C becomes equal to or less than the threshold value T1, the focus position is moved to the next position, and the evaluation value C is obtained again. The evaluation value C and the above-described count value may be stored in the memory 72 in association with each focus position. As a result of such processing, for example, in a case where the subject eye is opacified or the like, it is considered that the evaluation value C at the focus position transitions as illustrated in the graph of FIG. 7. Meanwhile, in a case where the subject eye is not opacified or the like, it is considered that the evaluation value C at the focus position transitions as illustrated in the graph of FIG. 8. Here, in the graph of FIG. 8, the gain is adjusted (reduced) once at the position of -4D.

For example, the target position of the focus position may be obtained based on the above-described count value and the evaluation value. In a case where the count values differ from each other between the focus positions, the target position is set from the focus positions having the largest count values. In a case where there are a plurality of focus positions having the largest count value, the position where the evaluation value becomes the maximum in the range may be set as the target position of the focus position. The estimated value of the maximum value obtained by performing the complement processing in the above-described range may be acquired as the target position of the focus position. In this manner, in the present example, the target position of the focus position can be obtained at once by performing the gain adjustment during the focus adjustment of the observation optical system 300 based on the observation image.

Further, here, for convenience of description, it is described that the target position of the focus position is obtained by using the count value of the number of times in which the gain is reduced, but the present invention is not necessarily limited thereto. At each focus position, the gain value is stored together with the evaluation value C, and the evaluation value C may be converted according to the gain value into a generalized value such that the evaluation values can be compared between different gains. The position where the generalized evaluation value is maximized can be obtained as the target position of the focus position. When generalizing, the evaluation value may be linearly converted (at least one of offset and variable power) depending on the gain value.

According to the above-described control, the initial value Gp of the gain is set to a sufficiently high value as described above, and thus, even in a case where the subject eye is opacified or the like, it is possible to sufficiently ensure a change in the evaluation value C of image quality with respect to a change in the focus position. Accordingly, even in a case where the subject eye is opacified or the like, it becomes easy to properly acquire the target position of the focus position. Further, in a case where the evaluation value C exceeds a threshold value T2, the gain is adjusted in a direction of lowering, and the evaluation value C is evaluated in consideration of the adjustment. Therefore, even in a case where the subject eye is not opacified, it is easy to properly acquire the target position of the focus position even though the initial value Gp of the gain is set to a high value to some extent.

Here, it has been described that the initial value Gp of the gain is set to a high value to some extent and the gain is reduced in a case where the evaluation value C exceeds the threshold value T1, but the present invention is not necessarily limited thereto. For example, the initial value of the gain may be set to a low value (a value lower than the above-described initial value Gp) to some extent, and the gain may be increased in a case where the evaluation value C is lower than the second threshold value T2. Even in this case, in order to reduce the saturation of the evaluation value C by changing the focus position, a control for reducing the gain in a case where the evaluation value C exceeds the threshold value T1 may be used in combination.

<Second Focus Adjustment>

Further, the control unit 70 moves the focusing lens 24 of the OCT optical system 200 to the same focus position as the focusing lens 63 (second focus adjustment). At this time, the control unit 70 moves the focusing lens 24 of the OCT optical system 200 based on the focus position of the observation optical system 300 obtained as a result of the first focus adjustment (autofocus on the OCT image).

For example, when the focus position of the observation optical system 300 is -3D, the focus position of the OCT optical system 200 is also controlled to be -3D. Each focus position in the observation optical system 300 and each focus position in the OCT optical system 200 may be associated with each other by, for example, diopter conversion.

When the position of the focusing lens 24 of the OCT optical system 200 is adjusted in this manner, an amount of return light from the fundus detected by the detector 83 increases.

Furthermore, the focus position information of the OCT optical system 200 may be acquired based on the OCT image, and the focusing lens 24 may be readjusted. For example, the position of the focusing lens 24 may be adjusted such that the focus position matches a predetermined layer. Accordingly, the focus position in the OCT optical system 200 can be adjusted more accurately.

<First Optical Path Length Adjustment>

The adjustment of the first optical path length will be described. The control unit 70 moves the mirror 31 and adjusts the position of the mirror 31 to a position where a tomographic image of the fundus is acquired based on the output signal output from the light receiving element 83 at each position of the mirror 31.

At this time, the control unit 70 moves the mirror 31 in one direction from the initial position by a predetermined step (for example, approximately several mm by the in-air conversion). The OCT image is acquired for each movement of the predetermined step, and the signal intensity of the OCT image is obtained.

In the present example, the signal intensity of the OCT image is represented as the value of evaluation value B. However, the present invention is not limited thereto, and various values that correlate with the signal intensity of the OCT image may be used as the evaluation value.

$$B = (\text{"average maximum luminance value of image"} - \text{"average luminance value of background region of image"})/\text{"standard deviation of luminance value of background region"}$$

The evaluation value B may be calculated from the entire image, or may be calculated based on the luminance information in a plurality of scanning lines in the depth direction.

As a result of the above-described control, the control unit 70 may store the position of the mirror 31 and the evaluation value B in the memory 75 in association with each other.

The evaluation value B at each position of the mirror 31 transitions as illustrated in the graph of FIG. 9, for example. In FIG. 9, the horizontal axis represents a position of the reference mirror, and the vertical axis represents the evaluation value B.

A position where the evaluation value B peaks is the adjustment target in the first optical path length adjustment. Thus, the control unit 70 obtains the position of the reference mirror at which the evaluation value B peaks, and adjusts the reference mirror to the obtained position.

Here, in the present example, since it is necessary to obtain a position where the evaluation value B peaks, for example, it is not necessary to move the mirror 31 over the entire movable range, and for example, the drive of the mirror 31 may be stopped at a position where an increase of the evaluation value B stops and the evaluation value B begins to decrease. Further, the control unit 70 may estimate a position of the reference mirror corresponding to the peak from the calculation result of the evaluation value B for each position of the mirror 31, and move the reference mirror to the estimated position as the adjustment target.

In general, the position of the mirror 31 when the real image of the fundus appears in the OCT image is a position where the peak of the evaluation value B is detected. However, in a case where there is out of focus, there is a case where the position of the mirror 31 in which a virtual image appears in the OCT image is a position where the peak of the evaluation value B is detected.

When the optical path length is roughly adjusted as described above, at least a part of the tomographic image of the fundus is displayed at any position on the monitor 72.

In the present example, since the first automatic optical path length adjustment and the focus adjustment are performed simultaneously, in a case where the focus adjustment is completed during the first automatic optical path length adjustment, the quality of the OCT image used for the first automatic optical path length is improved. Therefore, a case is considered where the evaluation value B changes and the peak detection position changes before and after the first automatic optical path length adjustment. Even in this case, in the first automatic optical path length adjustment, it is sufficient that at least a part of the tomographic image of the fundus is displayed on the monitor 72, and thus, the peak position does not necessarily have to be detected appropriately. That is, since it is sufficient that the optical path length is adjusted roughly, the peak detection accuracy does not necessarily have to be high.

<Second Optical Path Length Adjustment>

In the present example, after the focus adjustment and the first optical path length adjustment are completed, the control unit 70 moves the mirror 31 again in the optical axis direction to readjust (finely adjust) the OPL.

Here, the control unit 70 determines whether the image of the fundus included in the OCT image acquired after the focus adjustment is a real image or a virtual image. For example, the control unit 70 compares the full width at half maximum with respect to the peak in the luminance distribution in the depth direction in the OCT image with a predetermined threshold value. In a case where the full width at half maximum is smaller than the threshold value, the image of the fundus may be determined as a real image, and in a case where the full width at half maximum is equal to or greater than the threshold value, the image of the fundus may be determined as a virtual image. For the determination on whether the real image or the virtual image, any method may be used as long as the difference in image quality between the real image and the virtual image is used, and in addition to the full width at half maximum, for example, the contrast of the OCT image, the degree of rising of edge of the OCT image, and the like are used. Moreover, the shape of the OCT image may be used.

In a case where it is determined that the image of the fundus in the OCT image is the virtual image, the control unit 70 moves the mirror 31 in the direction in which the real image is acquired. Here, since a position where the real image is acquired has a correspondence relationship with a position where the virtual image is acquired, the adjustment may be performed as follows. That is, the position where the real image is acquired is in the direction in which the reference optical path is shorter than that at the position where the virtual image is acquired. Further, a movement amount of the mirror 31 that makes a deviation amount from a predetermined optical path length matching position S to the image detection position zero is calculated, and the mirror 31 may be moved with twice the movement amount as a movement amount from the position where the virtual image is acquired (to the position where the real image is acquired).

In a case where the image of the fundus in the OCT image is a virtual image, the method of adjusting the OPL to the position where the real image is acquired is not necessarily limited thereto.

In a case where it is determined that the image of the fundus in the OCT image is a real image, the control unit 70 adjusts the position of the real image. For example, the control unit 70 considers a position where the peak of the luminance distribution in the depth direction is detected as the image position, may calculate the displacement amount between a preset optical path length matching position S and the image position, and may move the mirror 31 such that the displacement amount disappears (refer to JP-A-2010-12111).

As described above, it is preferable that the control unit 70 performs the determination on whether the tomographic image of the fundus in the OCT image is real or virtual, and further the determination on whether or not the real image and the virtual image coexist in the OCT image simultaneously. At this time, for example, it may be determined whether or not the real image and the virtual image coexist based on the deviation amount from the optical path length matching position S to an image detection position PI.

For example, in a case where the image position P1 of the image of the fundus calculated as described above is in the vicinity of the upper end of the OCT image (for example, a region corresponding to 1/4 from the upper end of the OCT image), the control unit 70 may determine that the real image and the virtual image are in the coexisting state.

In this case, the control unit 70 may move the mirror 31 in a predetermined direction (direction in which the reference light becomes shorter) such that only the real image is acquired. The moving direction and the movement amount of the mirror 31 from the coexisting state of the real image and the virtual image to the state where only the real image is acquired may be predetermined experimentally or by simulation.

<Polarizer Adjustment>

In the present example, the control unit 70 drives the polarizer 33 to adjust the polarization state between the measurement light and the reference light. In a case where the polarization states match between the measurement light and the reference light, a stronger interference signal can be obtained. Here, the polarizer 33 is driven and controlled based on the output signal output from the light receiving element 83 such that the polarization states match between the measurement light and the reference light.

More specifically, in the present example, the polarizer 33 is driven and controlled based on the OCT image. While changing the position (orientation) of the polarizer 33, the control unit 70 obtains the signal intensity of the OCT image acquired each time the position changes. For example, the above-described evaluation value B may indicate the signal intensity. Polarizer adjustment is completed by obtaining the position of the polarizer 33 at which the evaluation value B (peak value) peaks and adjusting the position of the polarizer 33 to that position.

By completing the optimization control as described above, the fundus region desired by the examiner can be observed with high sensitivity and high resolution.

<Imaging OCT Image>

The description will be continued by returning to FIG. 2. As illustrated in FIG. 2, in the present example, when the examiner presses an imaging switch which is not illustrated after the optimization is completed, the OCT image is captured via the OCT optical system 200. The captured OCT image is stored in, for example, the memory 75.

<Modification Example>

Although the present disclosure has been described above based on the embodiments, the present disclosure is not necessarily limited thereto.

For example, in the above-described example, the images sequentially acquired by the imaging optical system (SLO optical system 300 in the above-described example) have been described as being one channel (one type) of only the observation image, but the present disclosure is not necessarily limited thereto. For example, there may be an imaging optical system capable of acquiring images of a plurality of channels at the same time. As an example, JP-A-2016-59399 by the present applicant discloses an SLO optical system capable of simultaneously acquiring a three-channel (three-color) fundus image. In a case where the plurality of channels of fundus images are sequentially acquired via such an optical system, the above-described <First Focus Adjustment>may be performed based on the fundus image of any one channel. Otherwise, the imaging condition related to the second optical device during the focus adjustment may be adjusted such that the luminance values of each of the fundus images of the plurality of channels are not saturated.

For example, in the above-described embodiment, it is described that, in any of the adjustment of the focus position in the observation optical system 300 (and the OCT optical system 200), and the adjustment of the imaging condition (amount of light, gain, and exposure time) related to the intensity of the light receiving signal in the observation optical system 300, the control is performed based on the information of the entire image in the observation image. However, the present disclosure is not limited thereto, and at least one of the controls may be performed based on the information of a part of the region in the observation image. For example, the evaluation values B and C in the above-described example may be acquired as statistics of a part of the region of the image. In this case, the adjustment can be performed more quickly.

Further, for example, when misalignment or the like occurs, there is a case where the illumination light to the fundus may be eclipsed on an iris. Accordingly, a case is considered where the validity of the above-described evaluation values is affected. Thus, for example, in the present embodiment, the validity of the evaluation value acquired at each focus position may be determined based on the image of the anterior eye part acquired by the anterior eye part observation optical system which is not illustrated.

Further, in the optimization control in the above-described example, except for a part (determination of suitability of the first optical path length adjustment), the previous adjustment is completed and then the next adjustment is performed. However, the present disclosure is not necessarily limited thereto, and the control unit 70 may determine whether or not the optimization adjustment is successful based on the luminance information of the OCT image, and stop the optimization adjustment based on the determination result. In a case where the control unit 70 determines that the adjustment has failed, the control unit 70 causes the optimization control to be repeated again. At this time, the optimization control may be stopped every time the optimization control fails, or the optimization control may be stopped in a case where the optimization control fails several times. Further, when the optimization fails, a display on which the optimization has failed may be displayed on the monitor 75 such that the examiner can select whether or not to perform the re-optimization.

Further, in the optimization control in the above-described example, the processing proceeded in the order of the first automatic optical path length adjustment, the focus adjustment, the second automatic optical path length adjustment, and the polarizer adjustment. However, the processing order is not necessarily limited thereto, and the processing order can be rearranged as appropriate. For example, the polarizer adjustment may be performed between completion of the first automatic optical path length adjustment and the focus adjustment and start of the second automatic optical path length adjustment.

In the present example, the focus adjustment may be performed before and after the second optical path length adjustment. In this case, in the first focus adjustment, the control unit 70 performs rough adjustment to the extent that fine adjustment of the optical path length by the second optical path length adjustment is possible, and completes the fine adjustment of the optical path length by the second optical path length adjustment, and then, in the second focus adjustment, the control unit 70 may match the focus.

In the present example, a configuration is employed in which the second automatic optical path length adjustment is started as soon as the focus adjustment is completed, but the present disclosure is not limited thereto. For example, a configuration may be employed in which the second automatic optical path length adjustment may be performed as soon as the focus adjustment and the first automatic optical path length adjustment are completed.

In the above description, various imaging conditions have been adjusted based on the OCT image, but the present disclosure is not necessarily limited thereto. For example, various imaging conditions may be adjusted based on data before imaging or the output signal from the detector before Fourier transform. Here, the OCT image, the data before imaging (the signal after Fourier transform), and the output signal from the detector are collectively referred to as OCT data. That is, various imaging conditions can be adjusted based on the OCT data.

REFERENCE SIGNS LIST

| | |
|---|---|
| 23 | scanning unit |
| 24 | focusing lens |
| 24a | driver |
| 31 | reference mirror |
| 63 | focusing lens |
| 63a | driver |
| 68 | light receiving element |
| 70 | control unit |
| 72 | memory |
| 75 | monitor |
| 83 | detector |
| 200 | OCT optical system |
| 300 | observation optical system |

**Claims**

1. An ophthalmologic imaging apparatus comprising:

an imaging optical system including an irradiation optical system that irradiates a subject eye with light and a light receiving optical system that receives return light from the subject eye by a light receiving element, and capturing an image of the subject eye based on a signal from the light receiving element;
a first optical device disposed in an optical path of the imaging optical system and controlled to adjust a focusing position of the imaging optical system with respect to the subject eye;
a second optical device controlled to adjust at least one imaging condition among an amount of the light, a gain for the signal from the light receiving element, and an exposure time; and
a control means for controlling the first optical device based on a sequentially-acquired image of the subject eye, and for controlling the second optical device based on the sequentially-acquired image of the subject eye simultaneously with or before controlling the first optical device.

**2.** The ophthalmologic imaging apparatus according to claim 1,
wherein the imaging optical system is a front imaging optical system that captures a front image of a fundus as the image of the subject eye.

**3.** The ophthalmologic imaging apparatus according to claim 2, comprising:

a second optical system that irradiates a captured scope of the front image with second light,
wherein a focusing position of the second optical system with respect to the subject eye is adjusted in conjunction with the focusing position of the imaging optical system.

**4.** The ophthalmologic imaging apparatus according to claim 3,
wherein the second optical system is an OCT optical system for acquiring an OCT image in the captured scope of the front image.

**5.** The ophthalmologic imaging apparatus according to claim 4, further comprising:

a third adjusting means for adjusting an OPL in the OCT optical system,
wherein the control means simultaneously controls the adjustment of the OPL together with the adjustment of the focusing position and the adjustment of the imaging condition.

**6.** The ophthalmologic imaging apparatus according to claim 2,
wherein the second optical system is an irradiation optical system that irradiates a captured scope of the front image with a therapeutic beam.

**7.** The ophthalmologic imaging apparatus according to claim 1,
wherein the imaging optical system is an OCT optical system that captures a tomographic image of a fundus as the image of the subject eye.

# FIG. 1

# FIG. 2

# FIG. 3

Z

# FIG. 4

START

↓

ANTERIOR EYE PART ALIGNMENT ADJUSTMENT

↓

OPTIMIZATION CONTROL

↓

IMAGE CAPTURING

↓

END

# FIG. 5

```
        ┌─────────────────────────────┐
        │   OPTIMIZATION CONTROL      │
        └─────────────────────────────┘
                      │
                      ▼
          ┌───────────────────────┐
          │    INITIALIZATION     │
          └───────────────────────┘
                      │
        ┌─────────────┴─────────────┐
        ▼                           ▼
┌──────────────────┐      ┌──────────────────────┐
│  FIRST OPTICAL   │      │    SIMULTANEOUS      │
│   PATH LENGTH    │      │ ADJUSTMENT OF FOCUS  │
│   ADJUSTMENT     │      │      AND GAIN        │
└──────────────────┘      └──────────────────────┘
        │                           │
        └─────────────┬─────────────┘
                      ▼
  ┌──────────────────────────────────────────┐
  │ SECOND OPTICAL PATH LENGTH ADJUSTMENT     │
  └──────────────────────────────────────────┘
                      │
                      ▼
     ┌────────────────────────────────────┐
     │     POLARIZATION ADJUSTMENT        │
     └────────────────────────────────────┘
                      │
                      ▼
        ┌─────────────────────────────┐
        │             END             │
        └─────────────────────────────┘
```

# FIG. 6

# FIG. 7

# FIG. 8

# FIG. 9

**INTERNATIONAL SEARCH REPORT**

| International application No. |
|---|
| PCT/JP2020/027126 |

A. CLASSIFICATION OF SUBJECT MATTER
A61B 3/10(2006.01)i
FI: A61B3/10 100; A61B3/10 300

According to International Patent Classification (IPC) or to both national classification and IPC

B. FIELDS SEARCHED

Minimum documentation searched (classification system followed by classification symbols)
A61B3/00-3/18

Documentation searched other than minimum documentation to the extent that such documents are included in the fields searched
| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2013-188316 A (NIDEK CO., LTD.) 26.09.2013 (2013-09-26) paragraphs [0010]-[0151], fig. 1-12 | 1-7 |
| Y | JP 2003-310556 A (TOPCON CORPORATION) 05.11.2003 (2003-11-05) paragraph [0073] | 1-7 |
| Y | JP 2016-193096 A (NIDEK CO., LTD.) 17.11.2016 (2016-11-17) paragraph [0013], fig. 1 | 6 |

☐ Further documents are listed in the continuation of Box C.   ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 03 September 2020 (03.09.2020) | 15 September 2020 (15.09.2020) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**
Information on patent family members

International application No.

PCT/JP2020/027126

| Patent Documents referred in the Report | Publication Date | Patent Family | Publication Date |
|---|---|---|---|
| JP 2013-188316 A | 26 Sep. 2013 | US 2013/0242258 A1 paragraphs [0020]- [0181], fig. 1-12 EP 2638849 A1 | |
| JP 2003-310556 A | 05 Nov. 2003 | US 2004/0004694 A1 paragraph [0107] | |
| JP 2016-193096 A | 17 Nov. 2016 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013188316 A
- JP 2010012111 A
- JP 2016059399 A